# EUROPÄISCHE PATENTSCHRIFT

(11) **EP 0 582 163 B1**
(45) Veröffentlichungstag und Bekanntmachung des Hinweises auf die Patenterteilung: **10.09.1997**
(21) Anmeldenummer: 93111908.5
(22) Anmeldetag: 24.07.1993
(51) Int. Cl.: A61M 5/00, A61M 39/02, A61M 5/165

(54) **Implantierbare Vorrichtung**
Implantable device
Dispositif implantable

(30) Priorität: 01.08.1992 DE 4225524
(43) Veröffentlichungstag der Anmeldung: 09.02.1994
(73) Patentinhaber: Fresenius AG, 61350 Bad Homburg (DE)
(72) Erfinder: Polaschegg, Hans-Dietrich, Dr., D-61440 Oberursel 4 (DE); Steinbach, Bernd, Dr., D-61341 Bad Homburg (DE)
(74) Vertreter: Fuchs, Luderschmidt & Partner Patentanwälte

(56) Entgegenhaltungen:
- DE-A- 3 915 251
- US-A- 5 108 377

## Beschreibung

Die Erfindung betrifft eine implantierbare Vorrichtung zur dosierten Abgabe von flüssigen Medikamenten in den menschlichen Körper mit einem Gehäuse, das wenigstens eine Kammer zur Speicherung des Medikaments aufweist, mit einer Einfüllöffnung im Gehäuse, das mittels eines durchstechbaren Septums verschlossen ist und mit der Kammer in Strömungsverbindung steht, einer Auslaßöffnung im Gehäuse, einem Katheterschlauch, der an die Auslaßöffnung angeschlossen ist, und mit einer Partikelrückhalteeinrichtung im Bereich der Auslaßöffnung.

Es sind implantierbare Imfusionseinrichtungen, sogenannte Infusionsports bekannt, bei denen der Implantationsort und der Verabreichungsort voneinander getrennt sind. Diese Ports bestehen aus einem üblicherweise unter die Haut implantierten Medikamentenreservoirs, das ein Gehäuse mit einem durchstechbaren Septum aufweist. Von einer im Gehäuse vorgesehenen Auslaßöffnung geht ein Katheterschlauch ab, dessen anderes Ende mit dem Verabreichungsort verbunden ist.

Infolgedessen besteht im Unterschied zu üblichen Kathetern bei den genannten implantierbaren Infusionseinrichtungen kein permanenter Durchbruch durch die Haut. Solche implantierbaren Infusionseinrichtungen, mit denen ein Bolus verabreicht werden kann, sind beispielsweise aus den deutschen Patentschriften 26 26 348, 35 18 841, EP 134 745 bekannt. Weiterhin sind implantierbare Infusionspumpen bekannt, deren Konstruktion im wesentlichen den eingangs genannten implantierbaren Infusionseinrichtungen entspricht, wobei diese Infusionspumpen ein Pumpmittel aufweisen, um das im Reservoir vorgelegte flüssige Medikament stetig durch den Katheter zu fördern. Üblicherweise wird die Kammer mittels einer gasundurchlässigen flexiblen Wand von einer weiteren Kammer getrennt, die ein isobar sich ausdehnendes Pumpmittel enthält. Derartige Infusionspumpen sind beispielsweise aus den US-Patentschriften 44 96 343 und 37 31 681, den deutschen Offenlegungsschriften 26 04 113 und 39 15 251 sowie der AT-A-391416 bekannt. Solche Infusionspumpen können neben einer Kammer, die stets mit Pumpmitteln beaufschlagt wird und die ihr Medikament durch eine Kapillare als durchflußregelndes Mittel abgibt, auch eine weitere Kammer mit einer weiteren Zuspritzöffnung, die mit einem Septum verschlossen ist, aufweisen, wobei diese Kammer ebenfalls mit der Auslaßöffnung verbunden, nicht jedoch von dem Pumpmittel beaufschlagt ist. Eine solche Vorrichtung ist beispielsweise aus der US-PS 44 96 343 und der DE-OS 39 15 251 bekannt.

Die Infusionseinrichtung wird an einer vorgesehenen Stelle unterhalb der Haut implantiert. Zur Infusion wird eine Hohlnadel (Kanüle) durch die Haut und das darunter liegende Septum der Infusionseinrichtung gestochen. Infolgedessen besteht die Gefahr, daß Partikel aus der Haut, dem Gewebe oder dem Septum in den Hohlraum des Infusionsports gelangen können und die Auslaßöffnung bzw. der Katheter durch diese Partikel verstopft werden. Deshalb wurde eine Reihe von mehr oder weniger aufwendigen Filtereinrichtungen vorgeschlagen, die eine solche Verstopfungsgefahr durch Partikel verhindern sollen. Solche Filtereinrichtungen sind in den vorstehend genannten Patentschriften erwähnt und bestehen in der Regel aus mehreren Teilen, die üblicherweise gegeneinander abgedichtet werden müssen.

Infolgedessen ist die Montage solcher Filtereinrichtungen aufwendig und somit kostspielig.

Eine implantierbare Vorrichtung zur dosierten Abgabe von flüssigen Medikamenten mit einer Partikelrückhalteeinrichtung ist ferner aus US-A-5,108,377 bekannt. Die Partikelrückhalteeinrichtung der bekannten Infusionsvorrichtung ist Bestandteil eines schaftförmigen Auslaßstückes, das in die Auslaßöffnung des Gehäuses der Infusionsvorrichtung passend eingesetzt ist. Sie wird durch einen im Zentrum der Auslaßöffnung angeordneten zylindrischen Ansatz des schaftförmigen Auslaßstückes gebildet, wodurch ein kompliziertes Bauteil entsteht.

Der vorliegenden Erfindung liegt die Aufgabe zugrunde, eine implantierbare Infusionseinrichtung der eingangs erwähnten Art zur Verfügung zus stellen, bei der die Partikelfilterprobleme auf einfache Weise gelöst werden.

Diese Aufgabe wird gelöst mit den Merkmalen des Anspruchs 1.

Vorteilhafterweise ist als verengendes Teil ein Draht oder eine Kapillare vom Innenraum der implantierbaren Vorrichtung aus in die Auslaßöffnung unter Bildung eines Ringspalts eingeführt, wobei der Draht oder die Kapillare selbst in der Kammerwand bzw. im Kammerboden verankert sind. Dabei wird die Auslaßöffnung üblicherweise durch das Lumen des Katheterschlauchs selbst gebildet, der innerhalb der im Gehäuse vorgesehenen Auslaßöffnung fest angeordnet ist. Insofern ragt der Draht oder die Kapillare in das Lumen des Katheterschlauchs hinein und bildet somit einen Ringspalt zwischen der Innenwand des Katheters und der Außenoberfläche des Drahts bzw. der Kapillare. Dabei hat der ringförmige Freiraum nur eine solche Ausdehnung, daß er sicher die Partikel zurückhält, die üblicherweise eine mittlere Größe kleiner 0,6 mm aufweisen.

Wie bereits erwähnt, dient eine Kapillare zur Strömungsregulierung bei solchen Infusionseinrichtungen, deren Medikamentenreservoir mit einer Pumpeinrichtung verbunden ist, die einen stetigen Druck auf das Reservoir ausübt. Infolgedessen ist der Kapillardurchmesser in Verbindung mit der Viskosität des flüssigen Medikaments bestimmend für die Durchflußrate der Flüssigkeit durch die Kapillare. Gemäß einer bevorzugten Ausführungsform wird eine solche Kapillare durch die Wand des Medikamentenreservoirs und eine zweite Zuspritzkammer hindurch direkt in das Lumen des Katheterschlauchs eingeführt.

Wie festgestellt, ist der Durchmesser des Ringspalts geringer als der Durchmesser eines Partikels, der in den ansonsten offenen Katheter gerissen werden könnte und diesen verstopfen könnte.

Es ist nicht erfindungswesentlich, daß der Draht zentrisch im Katheterlumen angeordnet ist. Vielmehr kann er auch seitlich des Zentrums versetzt angeordnet sein bzw. im Lumen floaten.

Gemäß einer weiteren Ausführungsform kann der Eingangsbereich mit einem Einsatzstück versehen sein, das unmittelbar in der Schlauch- bzw. Katheterinnenwand mittels Krallen verankert ist. Dieses Einsatzstück besteht im wesentlichen aus einem zylindrischen Zentralteil, von dem sich radial nach außen die genannten Krallen erstrecken. Dabei entspricht der zylindrische Zentralteil im wesentlichen dem drahtförmigen Einsatzstück, das in der vorstehend beschriebenen Ausführungsform erläutert worden ist. Insofern sind die Dimensionen des zylindrischen Einsatzstücks bzw. des zwischen dem Einsatzstück und dem Katheterschlauch gebildeten Ringspalts im wesentlichen identisch.

Weitere Ausführungsformen, Einzelheiten und Merkmale der Erfindung sind an zwei Beispielen unter Bezugnahme auf die Zeichnungen beschrieben.

Es zeigen
- Figur 1: eine Draufsicht auf eine erste Ausführungsform einer Infusionseinrichtung,
- Figur 2: einen Schnitt entlang der Linie II-II durch die in Figur 1 gezeigte Ausführungsform und
- Figur 3: einen Längsschnitt durch eine zweite Ausführungsform.

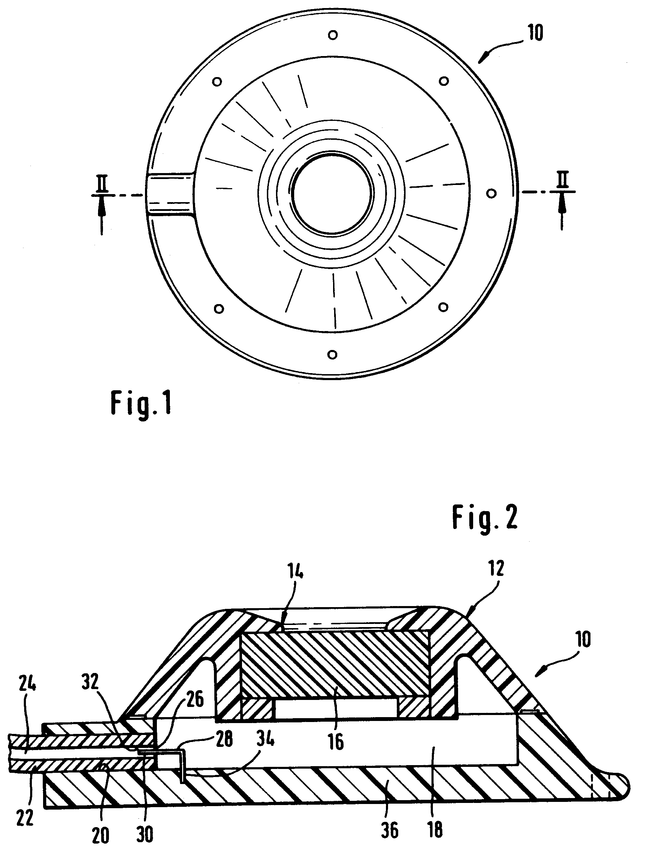

Mit 10 ist in Figur 1 eine Infusionseinrichtung bezeichnet, die im wesentlichen aus einem kapselförmigen Gehäuse 12 besteht, das eine Einfüllöffnung 14 aufweist, die mit einem durchstechbaren Septum 16 verschlossen ist. Im Gehäuse selbst ist eine Kammer 18 gebildet, die zur Speicherung eines flüssigen Medikaments dienen kann. Von der Kammer 18 geht eine Auslaßöffnung 20 ab, in die üblicherweise ein Katheterschlauch 22 eingeführt ist, der ein Lumen 24 aufweist. Derartige Infusionseinrichtungen sind bekannt und beispielsweise in den eingangs genannten Patentschriften beschrieben, insbesondere der EP 134 745, auf deren Offenbarung ausdrücklich Bezug genommen wird.

In den Eingangsbereich 26 des Lumens 24 ist ein Draht 28 mit seinem einen Ende 30 eingeführt, so daß zwischen der Außenoberfläche des Drahts 28 und der Innenwand des Katheterschlauchs 22, die das Lumen 24 begrenzt, ein Ringspalt 32 gebildet wird. Die Dicke des Ringspalts 32 liegt in einem Bereich von 50-250 µm.

Das andere Ende 34 des Drahts ist in dem Behälterboden 36 verankert, so daß hierdurch die Anordnung des Drahts 28 im Eingangsbereich 26 des Katheterschlauchs 22 festgelegt ist.

Der Behälter 12, das Septum 16 und der Katheterschlauch 22 bestehen üblicherweise aus medizinisch unbedenklichen Kunststoffen. Dabei ist die Herstellungsgenauigkeit des Katheterschlauchs 22 mit seinem Lumen 24 beschränkt, so daß nur Lumina mit einem Durchmesser > 0,2 mm mit Toleranzen von 10 % hergestellt werden können. Andererseits kann jedoch ein Draht sehr genau ausgezogen werden und hat somit bessere Fertigungstoleranzen.

Insofern können das Lumen 24 und der Draht 28 in ihrer Dimension relativ gut aneinander angepaßt werden. Deshalb kann also auch der Draht 28 zusätzlich als Strömungsregulierungsmittel dienen, was erfindungsgemäß jedoch nicht im Vordergrund steht. Insofern liegt die Funktion des Drahts 28 bei den relativ großen Katheterlumina 24 im wesentlichen bei der Filterwirkung, d.h. der Rückhaltewirkung von relativ großen Partikeln, die beim Durchstoßen der Haut und des Septums 16 mit einer Kanüle in die Kammer 18 eingeführt werden können. Diese Partikel können aufgrund der Anordnung des Drahts 28 im und vor dem Eingangsbereich 26 des Lumens 24 bzw. der Auslaßöffnung 20 diese Öffnungen nicht mehr verstopfen bzw. in diese Öffnungen eindringen und von dort in den Körper des Patienten gelangen.

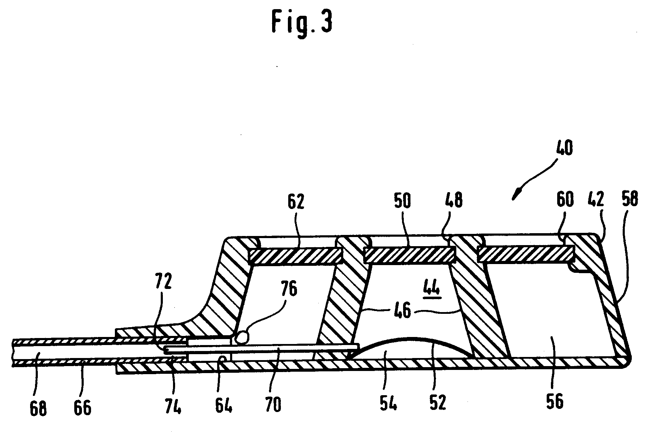

In Figur 3 ist eine Infusionspumpe 40 gezeigt, die zusätzlich zu der in Figuren 1 und 2 gezeigten Infusionseinrichtung 10 eine druckerzeugende Kammer aufweist.

Die implantierbare Infusionspumpe 40 weist ein Gehäuse 42 auf, das eine erste Kammer 44 aufweist, die durch die im wesentlichen hohlzylinderförmigen Seitenwände 46 begrenzt ist. Diese erste Kammer 44 weist eine erste Einfüllöffnung 48 auf, die durch ein erstes Septum 50 verschlossen ist.

Die erste Kammer 44 ist durch eine gasundurchlässige bewegliche Membran 52 von einer Pumpkammer 54 getrennt, in der ein sich isobar ausdehnendes gasförmiges/flüssiges Pumpmittel, üblicherweise Fluorkohlenwasserstoffe, vorgesehen ist. Um die im wesentlichen zylinderförmigen Seitenwände 46 herum ist eine zweite ringförmige Kammer 56 vorgesehen, die durch die im wesentlichen zylinderförmige Außenwand des Gehäuses 58 begrenzt ist. Diese Kammer 56 weist eine ringförmige Einlaßöffnung 60 auf, die durch ein ringförmiges zweites Septum 62 verschlossen ist.

Von der zweiten Kammer 56 geht eine Auslaßöffnung 64 durch die Außenwand 58 des Gehäuses 42 nach außen ab. In diese Auslaßöffnung 64 ist ein Katheterschlauch 66 mit einem Lumen 68 dicht eingeführt.

Demzufolge steht die zweite Kammer 56 über das Lumen 68 unmittelbar mit dem Verabreichungsort im Patienten in Strömungsverbindung. Andererseits steht die erste Kammer 44 über ein Kapillarrohr 70, das die Seitenwand 46 durchsetzt, direkt mit dem Lumen 68 in Strömungsverbindung. Wie aus Figur 3 ersichtlich ist, durchquert die als Strömungsbegrenzungsmittel dienende Kapillare 70 die ringförmige zweite Kammer 56 sowie den nicht vom Katheterschlauch 66 belegten Bereich der Auslaßöffnung 64 und ist mit seinem Ende 72 um einen bestimmten Betrag in das Lumen 68 des Katheterschlauchs 66 eingeführt. Dabei ist der Außendurchmesser der Kapillare 70 sowie der Durchmesser des Lumens 68 den Abmessungen des Drahts 28 bzw. des Lumens 24 gemäß der ersten Ausführungsform vergleichbar.

Es muß nicht hinzugefügt werden, daß es auch ausreicht, wenn der Draht 28 bzw. die Kapillare 70 nur in die Auslaßöffnung 20 bzw. 64 eingeführt ist, sofern sich die vorstehend genannten Abmessungen des sich hierdurch bildenden Ringspalts ergeben.

Die in Figur 3 dargestellte Infusionspumpe 40 verfügt somit über zwei Medikamentenreservoire 44 und 56, die separat befüllt werden können. So wird vom ersten Reservoir, d.h. der ersten Kammer 44, stetig durch die Kapillare 70 durch die Wirkung des in der Pumpkammer 54 vorgesehenen Pumpmittels ein Medikament direkt in den Katheter 66 abgegeben. Soll jedoch ein Bolus eines zweiten Medikaments zugespritzt werden, so wird dieser durch das Septum 62 mit einer Nadel zugeführt, wodurch die erfindungsgemäß zurückzuhaltenden Partikel Probleme erzeugen können. Hier verhindert die Kapillare 70 infolge des Ringspalts 74 zwischen Kapillare 70 und Katheterschlauch 66 bzw. der Einlaßöffnung 64 ein Einschleppen der ausgestanzten Partikel, wie mit 76 dargestellt, in den Katheterschlauch 66. Erfindungswesentlich an dieser Ausführungsform ist also die Einführung der Kapillare 70 in die Auslaßöffnung 64 bzw. das Lumen 68 des Katheterschlauchs.

## Patentansprüche

1. Implantierbare Vorrichtung zur dosierten Abgabe von flüssigen Medikamenten in den menschlichen Körper mit einem Gehäuse (12), das wenigstens eine Kammer (18) zur Speicherung eines flüssigen Medikaments aufweist, einer Einfüllöffnung (14) im Gehäuse, die mittels eines durchstechbaren Septums (6) verschlossen ist und mit der Kammer (18) in Strömungsverbindung steht, mit einer Auslaßöffnung (20, 64; 24, 68) im Gehäuse, mit einem Katheterschlauch (22), der an die Auslaßöffnung angeschlossen ist und mit einer Partikelrückhalteeinrichtung (28, 70) im Bereich der Auslaßöffnung, die den Querschnitt der Auslaßöffnung unter Bildung eines Ringspaltes (32, 74) verengt, **dadurch gekennzeichnet**, daß die Partikelrückhalteeinrichtung ein drahtförmiges Teil (28) oder eine Kapillare (70) ist, wobei das drahtförmige Teil bzw. die Kapillare aus der Kammer (18) unter Bildung des Ringspaltes (32, 74) in die Auslaßöffnung (20, 64; 24, 68) hineinragt und in der Kammerwand (36; 46) verankert ist.

2. Implantierbare Vorrichtung nach Anspruch 1, **dadurch gekennzeichnet**, daß das den Querschnitt verengende Teil ein sternförmiges Ankerteil ist, das einen zylinderförmigen Zentralteil aufweist, von dem sich radial mehrere Krallen erstrecken, die sich in der Gebrauchslage in der Innenwand der Auslaßöffnung oder des Lumens (20, 64; 24, 68) des Katheterschlauchs (22) verkrallen.

3. Vorrichtung nach einem der Ansprüche 1 bis 2, **dadurch gekennzeichnet**, daß die Dicke des Ringspaltes (32, 74) kleiner ist als der mittlere Durchmesser eines Partikels.

## Claims

1. Implantable device for dosed administration of liquid medicines to the human body with a housing (12), which has at least one chamber (18) to store a liquid medicine, an inlet aperture (14) in the housing, which is sealed by a perforatable septum (6) and whose flow is connected to the chamber (18), with an outlet aperture (20, 64; 24, 68) in the housing, with a catheter tube (22) which is connected to the outlet aperture and with a particle retention device (28, 70) in the area of the outlet aperture restricting the cross section of the outlet aperture by forming a ring slot (32, 74), **characterised by the fact that** the particle retention device is a wire-like section (28) or a capillary (70), whereby the wire-like section or the capillary protrudes from the chamber (18) into the outlet aperture (20, 64; 24, 68) to form the ring slot (32, 74) and is anchored in the chamber wall (36;46).

2. Implantable device as per Claim 1, **characterised by the fact that** the section restricting the cross section is a star-shaped anchor piece with a cylindrical central section, from which several claws extend radially and in the operative mode hook on to the inner wall of the outlet aperture or the lumen (20, 64; 24, 68) of the catheter tube (22).

3. Device as per one of claims 1 to 2, **characterised by the fact that** the width of the ring slot (32, 74) is smaller than the average diameter of a particle.

## Revendications

1. Dispositif implantable pour la distribution dosée de médicaments liquides dans le corps humain pourvu d'un boîtier (12), qui présente au moins une chambre (18) pour le stockage d'un médicament liquide, d'une ouverture de remplissage (14) ménagée dans le boîtier, qui est fermée au moyen d'un septum (6) transperçable et est en liaison d'écoulement avec la chambre (18), d'une ouverture de sortie (20, 64 ; 24, 68) pratiquée dans le boîtier, d'un tuyau de cathéter (22), qui est raccordé à l'ouverture de sortie et d'un dispositif de retenue des particules (28, 70) dans la zone de l'ouverture de sortie, qui rétrécit la section de l'ouverture de sortie en formant une fente annulaire (32, 74), caractérisé en ce que le dispositif de retenue de particules est une partie (28) filiforme ou un capillaire (70), la partie filiforme ou le capillaire sortant de la chambre (18) dépassant à l'intérieur de l'ouverture de sortie (20, 64 ; 24, 68) en formant la fente annulaire (32, 74) et étant ancré dans la paroi de chambre (36 ; 46).

2. Dispositif implantable selon la revendication 1, caractérisé en ce que la partie rétrécissant la section est une pièce d'ancrage en forme d'étoile qui présente une partie centrale cylindrique de laquelle s'étendent dans un plan radial plusieurs griffes qui s'accrochent dans la position d'utilisation dans la paroi interne de l'ouverture de sortie ou du lumen (20, 64 ; 24, 68) du tuyau de cathéter (22).

3. Dispositif selon l'une quelconque des revendications 1 ou 2, caractérisé en ce que l'épaisseur de la fente annulaire (32, 74) est inférieure au diamètre moyen d'une particule.
